# EUROPÄISCHE PATENTSCHRIFT

(11) **EP 1 948 310 B1**
(45) Veröffentlichungstag und Bekanntmachung des Hinweises auf die Patenterteilung: **11.03.2009**
(21) Anmeldenummer: 06818405.0
(22) Anmeldetag: 07.11.2006
(51) Int. Cl.: A61N 5/06, G02B 1/06, F21V 9/12

(54) **BESTRAHLUNGSVORRICHTUNG MIT FILTERSCHEIBEN UND DAZWISCHEN EINGESCHLOSSENEM MEDIUM**
IRRADIATION DEVICE WITH FILTER DISKS AND MEDIUM ENCLOSED THEREBETWEEN
DISPOSITIF D IRRADIATION AVEC PLAQUES FILTRANTES ET SUPPORT CONFINÉ ENTRE CELLES-CI

(30) Priorität: 16.11.2005 DE 202005017895 U
(43) Veröffentlichungstag der Anmeldung: 30.07.2008
(73) Patentinhaber: Maxs AG, 6072 Sachseln (CH)
(72) Erfinder: BRAUN, Werner, CH-6062 Wilen-Sarnen (CH); LÖFFLER, Dietmar, 79238 Ehrenkirchen (DE)
(74) Vertreter: Grünecker, Kinkeldey, Stockmair & Schwanhäusser Anwaltssozietät
(86) Internationale Anmeldenummer: PCT/EP2006/010662
(87) Internationale Veröffentlichungsnummer: WO 2007/057110

(56) Entgegenhaltungen:
- EP-A- 0 485 864
- EP-A- 0 678 705
- DE-A1- 19 515 182
- DE-C- 911 525
- DE-C1- 4 042 259
- GB-A- 1 084 335

## Beschreibung

Die Erfindung betrifft eine Bestrahlungsvorrichtung mit einer Strahlenquelle und einem im Strahlengang angeordneten Filter, der zwei transparente, im Wesentlichen planparallel zu einander angeordnete Filterscheiben aufweist, die mit ihren umlaufenden Ränder in einem Rahmen aus gut wärmeleitendem Material gehalten sind, wobei die Filterscheiben und der Rahmen einen geschlossenen Hohlraum begrenzen, in dem ein das Strahlenspektrum selektiv beeinflussendes Medium vorgesehen ist, wobei der Hohlraum als Druckkammer ausgebildet ist, so dass im Betrieb der Bestrahlungsvorrichtung Überdruck in dem Hohlraum herrscht und die Filterscheiben aus Glaskeramik oder Mineralglas bestehen.

Derartige Bestrahlungsvorrichtungen, die zur Wärmetherapie des menschlichen Körpers eingesetzt werden können, sind schon seit längerem bekannt. Die DE 911525 C offenbart eine gattungsgemäße Vorrichtung. In der EP 0 678 705 B1 wird ebenfalls eine Bestrahlungsvorrichtung mit einer Strahlenquelle und einem im Strahlengang angeordneten Filter beschrieben. Da nicht das gesamte, von der Strahlenquelle ausgesandte Strahlenspektrum für die Wärmetherapie geeignet ist, wird ein geeigneter Filter ausgewählt, der bestimmte Banden aus dem Strahlenspektrum herausfiltert. Der Filter besteht aus einem Rahmen, in dem zwei Filterscheiben angeordnet sind. Durch die Filterscheiben und den Rahmen wird ein Hohlraum gebildet, in dem sich ein Medium befindet, das das Strahlenspektrum selektiv beeinflusst. Im Betrieb der Bestrahlungsvorrichtung erwärmen sich die Filterscheiben und das im Hohlraum eingeschlossene Medium durch die von der Strahlenquelle produzierte Wärme. Durch die Erwärmung dehnt sich das in dem Hohlraum befindliche Medium aus und übt Druck auf die Filterscheiben aus. Da mindestens eine der Filterscheiben üblicherweise aus Kunststoff ausgebildet ist, kann sich diese verformen, wodurch die Filtereigenschaften beeinflusst würden. Die Bestrahlungsvorrichtung weist daher eine Druckausgleichsvorrichtung auf, die Druckschwankungen in dem Hohlraum ausgleicht. Bei der Erwärmung des Mediums in dem Hohlraum zwischen den Filterscheiben kann es weiterhin vorkommen, dass in dem Medium gelöste Gase ausgasen und Gasblasen die Filterwirkung des Filters beeinflussen. Bei der Bestrahlungvorrichtung der vorliegenden Erfindung wird jedoch ein Ausgasen des Mediums bei Erwärmung verhindert und werden die Filterscheiben bei Druckanstieg nicht verformt.

Es ist die Aufgabe der vorliegenden Erfindung eine Gefährdung des Patienten durch unzulässige Druckerhöhung im Hohlraum zwischen den Filterscheiben aus zu schliessen.

Hierzu ist erfindungsgemäß vorgesehen, dass die näher an der Strahlenquelle angeordnete Filterscheibe als Sicherheitsvorrichtung ausgebildet ist, so dass sie bruchanfälliger ist als die Filterscheibe, die weiter von der Strahlenquelle entfernt ist. Durch den Druckaufbau im Hohlraum zwischen den Filterscheiben kann somit dem Entgasen der Flüssigkeit bei einer Temperaturerhöhung entgegen gewirkt werden. Durch den Einsatz von Materialien wie Glaskeramik oder Mineralglas kann vermieden werden, dass sich die Filterscheiben durch den Druck im Hohlraum verformen, wodurch ein Linseneffekt entstehen würde, der die Filtereigenschaften beeinflusst. Durch die erfindungsgemäße Ausbildung der Filterscheiben wird sichergestellt, dass im Fall einer unzulässigen Druckerhöhung im Hohlraum zwischen den Filterscheiben die innere, näher an der Strahlenquelle angeordnete Filterscheibe zuerst bricht, wodurch eine Gefährdung des Patienten ausgeschlossen wird. Bei der DE 911525 C wird zum selben Zweck eine weitere Glasplatte auf der dem Patienten zugekehrten Seite des Filters angebracht. Die DE 19515182 A offenbart eine ähnliche Lösung, während die DE 4042259 C und die GB 1,084,335 Thermoschalter oder Druckwächter zur Sicherheit offenbaren.

Weiterhin ist aus dem bereits ermähnten britischen Patent GB 1,084,335 bereits eine Beleuchtungseinrichtung bekannt, die eine aus zwei voneinander beabstandete Glasplatten bestehende Frontabdeckung aufweist Der Hohlraum in der Frontabdeckung ist mit einem ebenfalls einen Hohlraum aufweisenden Reflektorschirm der Beleuchtungseinrichtung verbunden. In diese Hohlräume wird unter Druck Wasser eingefüllt, das kontinuierlich zirkuliert, um die Beleuchtungseinrichtung zu kühlen. Aus dieser Erfindung ist aber weder bekannt, dass mit Hilfe eines geeigneten Mediums bestimmte Banden aus dem Strahlenspektrum der Strahlenquelle herausgefiltert werden können, noch ist eine Sicherheitsvorrichtung für den Fall eines unzulässigen Druckaufbaus in den Hohlräumen beschrieben.

In einer weiteren vorteilhaften Ausführungsform der vorliegenden Erfindung wird beschrieben, dass die näher an der Strahlenquelle angeordnete Filterscheibe eine Sollbruchstelle aufweist. Auf diese Weise kann sichergestellt werden, dass die innere, von dem Patienten abgewandte Filterscheibe im Falle einer unzulässigen Druckerhöhung im Hohlraum zuerst zerbricht. Somit kann eine Verletzung des Patienten ausgeschlossen werden.

Gemäß einer weiteren Variante vorgesehen, dass die näher an der Strahlenquelle angeordnete Filterscheibe dünner ist als die Filterscheibe, die weiter von der Strahlenquelle entfernt ist Auch in dieser Variante wird sichergestellt, dass die innere Filterscheibe zuerst bricht, falls sich der Druck in dem Hohlraum zwischen den Filterscheiben unzulässig erhöht. In dieser Variante sind zudem keine zusätzlichen Bearbeitungsschritte der inneren Filterplatte erforderlich.

Gemäß einer weiteren Variante kann auch vorgesehen werden, dass die näher an der Strahlenquelle angeordnete Filterscheibe aus einem bruchanfälligeren Material besteht, als die Filterscheibe, die weiter von der Strahlenquelle entfernt ist. Auch hier wird wieder erreicht, dass die innere Filterscheibe im Fall eines Druckanstiegs in dem Hohlraum zwischen den Filterscheiben zuerst springt. Zusätzlich können den beiden Filterscheiben durch die unterschiedlichen Materialien unterschiedliche optische Eigenschaften verliehen werden.

Gemäß einer weiteren Ausführungsform ist vorgesehen, dass der Rahmen der Bestrahlungsvorrichtung an seinem Außenumfang Kühlrippen aufweist. Durch die Kühlrippen wird die beim Betrieb der Bestrahlungsvorrichtung entstehende Wärme abgeführt, so dass sich das in dem Hohlraum zwischen den Filterscheiben befindliche Medium weniger stark erwärmt und somit auch weniger stark zum Ausgasen neigt.

Vorteilhafterweise kann die Bestrahlungseinrichtung einen Lüfter umfassen und die Kühlrippen können so ausgebildet sein, dass der freie Querschnitt zwischen den Kühlrippen und einem Gehäuse der Bestrahlungseinrichtung in Bezug auf die Luftleistung des Lüfters optimiert ist. Dadurch wird erreicht, dass der vom Lüfter geförderte Luftstrom beim Durchgang durch den freien Querschnitt zwischen den Kühlrippen nicht beschleunigt wird. Auf diese Weise kann die Kühlung der Filtereinrichtung optimiert werden. Zusätzlich wird die Geräuschbildung verringert.

Im Folgenden wird eine Ausführungsform der Erfindung anhand einer Zeichnung näher erläutert.

Es zeigen:
- Fig. 1: Schnitt durch eine Bestrahlungsvorrichtung,
- Fig. 2: Draufsicht auf das Filterelement mit Rahmen und
- Fig. 3: Schnitt durch das Filterelement mit Rahmen entlang der Linie B-B in Fig. 2.

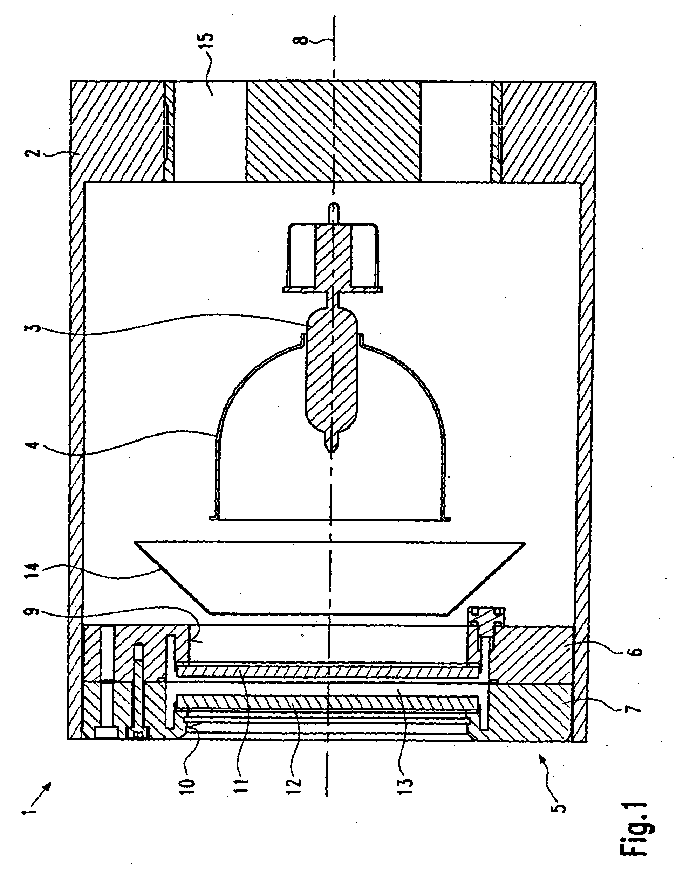

Fig. 1 zeigt einen Schnitt durch die Bestrahlungsvonichtung 1. Die Bestrahlungsvorrichtung umfasst ein Gehäuse 2, in dem eine Strahlenquelle 3 angeordnet ist. Als Strahlenquelle 3 kann beispielsweise eine Halogenlampe eingesetzt werden. Benachbart zu der Strahlenquelle 3 ist ein Reflektor 4 angeordnet. Bevorzugt wird ein Halbkugelreflektor eingesetzt, mit dem ein sehr homogenes Bestrahlungsfeld erzielt werden kann. Im Strahlengang der Strahlenquelle 3 ist das Filterelement 5 angeordnet. Das Filterelement 5 umfasst einen Rahmen, der aus einem näher an der Strahlenquelle angeordneten Rahmenteil 6 und einem weiter von der Strahlenquelle entfernten Rahmenteil 7 besteht Die beiden Rahmenteile 6, 7 weisen je eine relativ große zu der Filterachse 8 konzentrische Bohrung 9, 10 auf. In jeder der Bohrungen 9, 10 ist eine Filterscheibe 11, 12 angeordnet. Die Filterscheiben 11, 12 sind in den jeweiligen Rahmenteilen 6, 7 mit axialem Abstand zueinander angebracht, so dass zwischen ihnen ein scheibenförmiger Hohlraum 13 entsteht Der Hohlraum 13 ist mit einem das Strahlenspektrum selektiv beeinflussendem Medium gefüllt, um bestimmte Banden aus der von der Strahlenquelle 3 abgegebenen Strahlung heraus zu filtern. Üblicherweise besteht dieses Medium bei Filtern zum Einsatz bei der Wärmetherapie aus Wasser, dem eventuell noch Fungizide beigesetzt sind.

Zwischen der Strahlenquelle 3 und dem Filterelement 5 ist ein zur Filterachse 8 konzentrisches Hitzeschild 14 angeordnet. Dieses Hitzeschild 14 schirmt das Filterelement 5 von Strahlung ab, die sonst auf das Filterelement 5 treffen und dieses zusätzlich aufheizen würde.

Als weitere Kühleinrichtung ist auf der dem Filterelement entgegengesetzten Seite der Strahlenquelle 3 ein Lüfter 15 angeordnet

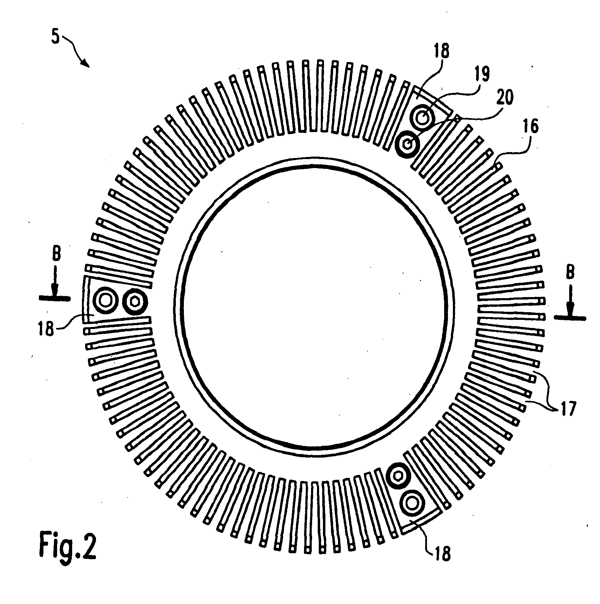

In Fig. 2 ist eine Draufsicht auf das Filterelement 5 der Bestrahlungsvorrichtung 1 dargestellt. Die beiden Rahmenhälften 6, 7 des Filterelements 5 weisen an ihren Rändern lamellenförmige Kühlrippen 16 auf. Die Kühlrippen 16 sind so ausgebildet, dass der zwischen ihnen entstehende freie Querschnitt 17 in Bezug auf die Lüftleistung des Lüfters 15 optimiert ist. Dadurch kann der vom Lüfter 15 geförderte Luftstrom den zwischen den Kühlrippen 16 gebildeten freien Querschnitt 17 durchströmen, ohne dabei beschleunigt zu werden. Somit werden Störgeräusche vermieden und eine optimale Kühlung des Filterelements 5 erreicht.

In regelmäßigen Abständen sind anstelle der Kühlrippen verbreiterte Stege 18 in den beiden Rahmenteilen 6 und 7 angeordnet. In diesen Stegen 18 sind parallel zur Filterachse 8 verlaufende Längsbohrungen 19, 20 angebracht, die Befestigungsmittel zum Verspannen der Rahmenteile 6 und 7 gegeneinander, sowie Befestigungsmittel zur Befestigung des Filterelements 5 im Gehäuse 2 der Bestrahlungsvorrichtung 1 aufnehmen können.

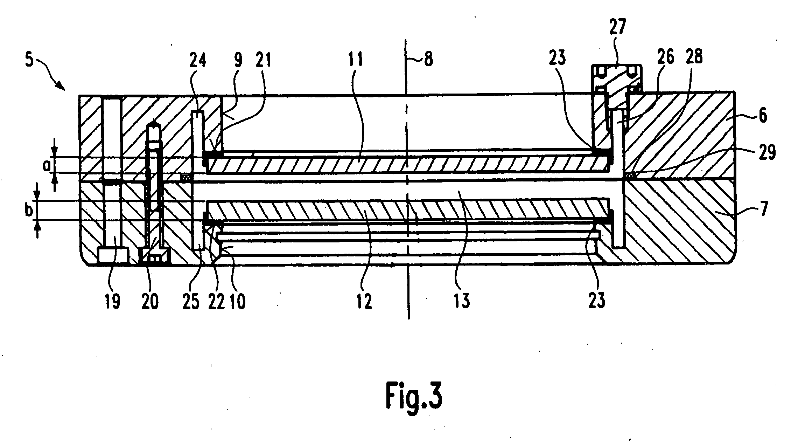

In Fig. 3 ist ein Schnitt durch das in Fig. 2 dargestellte Filterelement 5 entlang der Linie B-B dargestellt Die beiden Rahmenhälften 6, 7 weisen einen verbreiterten Steg 18 mit Längsbohrungen 19, 20 auf, in denen Befestigungsmittel angebracht werden können, um die Rahmenhälften 6, 7 gegeneinander zu verspannen und im Gehäuse 2 der Bestrahlungsvorrichtung 1 zu befestigen. Benachbart zu den großen, konzentrisch zu der Filterachse 8 angeordneten Durchgangsbohrungen 9, 10 ist in jeder der Rahmenhälften 6, 7 eine Stufe 21, 22 angebracht. Die Filterscheiben 11, 12 sind so in die Rahmenhälften 6, 7 eingebracht, dass ihre umlaufenden Ränder auf den Stufen 21, 22 aufliegen. Eine Befestigung der Filterscheiben 11, 12 in den Rahmenhälften 6, 7 kann beispielsweise mit einem Klebstoff 23 erfolgen. Die näher an der Strahlenquelle 3 angeordnete Filterscheibe 11 ist als Sicherheitsvorrichtung ausgebildet. Im Falle eines unzulässigen Druckanstiegs im Hohlraum 13, der beispielsweise durch einen Ausfall des Lüfters 15 bedingt wird, wird also zuerst die Filterscheibe 11 zerstört. Die dem Patienten zugewandte Filterscheibe 12 bleibt intakt, um Verletzungen zu vermeiden. Zu diesem Zweck ist die Dicke a der Filterscheibe 11 geringer als die Dicke b der Filterscheibe 12. Es ist auch denkbar, dass die Filterscheibe 11 eine Sollbruchstelle aufweist oder aus weniger druckbeständigen Material besteht als die Filterscheibe 12. Als Material für die Filterscheiben 11, 12 kann Glaskeramik oder Borosilikatglas eingesetzt werden.

Benachbart zu der Stufe 21, 22 weist jedes Rahmenteil 6, 7 eine Ringnut 24, 25 auf. Diese Ringnuten 24, 25 stehen mit dem Hohlraum 13 zwischen den Filterscheiben 11 und 12 in Verbindung und vergrößern diesen. Eine dieser Ringnuten 24, 25 weist eine Bohrung 26 auf, über die das Medium in den Hohlraum eingeführt werden kann. Die Bohrung 26 kann mit einem geeigneten Stopfen 27 verschlossen werden.

Benachbart zu der Ringnut 24, 25 ist in einem der Rahmenteile 6, 7 eine weitere Ringnut 28 angeordnet. In dieser Ringnut 28 wird ein Dichtungsmittel 29, wie beispielsweise ein O-Ring angebracht, um die beiden Rahmenteile gegeneinander abzudichten.

Im Betrieb der Bestrahlungsvorrichtung 1 gibt die Strahlenquelle 3 Strahlung ab, die über den Reflektor 4 in Richtung des im Strahlengang angeordneten Filterelements 5 gelenkt wird. Durch die dabei entstehenden Wärme erwärmt sich das im Hohlraum 13 befindliche Medium und will sich ausdehnen, wodurch der Druck im Hohlraum 13 ansteigt. Um eine Verformung der Filterscheiben 11, 12 und die dabei entstehende Linsenwirkung der Filterscheiben zu vermeiden, bestehen die Filterscheiben 11, 12 vorzugsweise aus einem starrem Material, wie Glaskeramik oder Borosilikatglas. Durch den erhöhten Druck kann ein Ausgasen des im Hohlraum 13 befindlichen Mediums vermieden bzw. verringert werden. Es entstehen in dem Hohlraum 13 also keine Gasblasen, die die Filterwirkung beeinflussen könnten.

Übersteigt der Druck in dem Hohlraum 13 beispielsweise aufgrund eines defekten Lüfters 15 einen kritischen Wert, so platzt die der Strahlenquelle 3 zugewandte dünnere Filterscheibe 11, da diese aufgrund ihrer Gestaltung bruchanfälliger ist als die Filterscheibe 12, die weiter von der Strahlenquelle 3 entfernt ist. So wird verhindert, dass trotz des im Betrieb erwünschten hohen Drucks im Problemfall Glasscherben auf den Patienten treffen oder anderweitig unkontrolliert herumfliegen; sie bleiben vielmehr im Gehäuse 2 der Bestrahlungsvorrichtung 1.

## Patentansprüche

1. Bestrahlungsvorrichtung (1) mit einer Strahlenquelle (3) und einem im Strahlengang angeordneten Filter (5), der zwei transparente, im Wesentlichen planparallel zueinander angeordnete Filterscheiben (11, 12) aufweist, die mit ihren umlaufenden Rändern in einem Rahmen (6, 7) aus gut wärmeleitendem Material gehalten sind, wobei die Filterscheiben (11, 12) und der Rahmen (6, 7) einen geschlossenen Hohlraum (13) begrenzen, indem ein das Strahlenspektrum selektiv beeinflussendes Medium vorgesehen ist, wobei der Hohlraum (13) als Druckkammer ausgebildet ist, so dass im Betrieb der Bestrahlungsvorrichtung (1) Überdruck in dem Hohlraum (13) herrscht und die Filterscheiben (11, 12) aus Glaskeramik oder Mineralglas bestehen, **dadurch gekennzeichnet,** die näher an der Strahlenquelle (3) angeordnete Filterscheibe (11) als Sicherheitsvorrichtung ausgebildet ist, so dass sie bruchanfälliger ist als die Filterscheibe (12), die weiter von der Strahlenquelle (3) entfernt ist.

2. Bestrahlungsvorrichtung (1) nach Anspruch 1, **dadurch gekennzeichnet, dass** die näher an der Strahlenquelle (3) angeordnete Filterscheibe (11) eine Sollbruchstelle aufweist.

3. Bestrahlungsvorrichtung (1) nach Anspruch 1 oder 2, **dadurch gekennzeichnet, dass** die näher an der Strahlenquelle (3) angeordnete Filterscheibe (11) dünner ist, als die Filterscheibe (12), die weiter von der Strahlenquelle (3) entfernt ist.

4. Bestrahlungsvorrichtung (1) nach einem der Ansprüche 1 bis 3, **dadurch gekennzeichnet, dass** die näher an der Strahlenquelle (3) angeordnete Filterscheibe (11) aus einem bruchanfälligeren Material besteht, als die Filterscheibe (12), die weiter von der Strahlenquelle (3) entfernt ist.

5. Bestrahlungsvorrichtung (1) nach einem der Ansprüche 1 bis 4, **dadurch gekennzeichnet, dass** der Rahmen (6, 7) an seinem Außenumfang Kühlrippen (16) aufweist.

6. Bestrahlungsvorrichtung (1) nach einem der Ansprüche 1 bis 5, **dadurch gekennzeichnet, dass** die Bestrahlungsvorrichtung (1) einen Lüfter (15) umfasst und die Kühlrippen (16) so ausgebildet sind, dass der freie Querschnitt (17) zwischen den Kühlrippen (16) und einem Gehäuse (2) der Bestrahlungsvorrichtung (1) in Bezug auf die Luftleistung des Lüfters (15) optimiert ist.

## Claims

1. An irradiation device (1) having a radiation source (3) and a filter (5) which is arranged in the optical path and has two transparent filter disks (11, 12) which are substantially arranged in plane-parallel configuration relative to each other and which are held with their surrounding edges in a frame (6, 7) made of a material of good heat conduction, the filter disks (11, 12) and the frame (6, 7) defining a closed cavity (13) which has provided therein a medium which selectively influences the radiation spectrum, the cavity (13) being configured in the form of a pressure chamber so that during operation of the irradiation device (1) overpressure prevails in the cavity (13) and the filter disks (11, 12) being made of glass ceramic or mineral glass, **characterized in that** the filter disk (11) which is arranged closer to the radiation source (3) is configured as a safety device so that it is more susceptible to breaking than the filter disk (12) which is further away from the radiation source (3).

2. The irradiation device (1) according to claim 1, **characterized in that** the filter disk (11) arranged closer to the radiation source (3) has a predetermined breaking point.

3. The irradiation device (1) according to claim 1 or 2, **characterized in that** the filter disk (11) arranged closer to the radiation source (3) is thinner than the filter disk (12) located further away from the radiation source (3).

4. The irradiation device (1) according to any one of claims 1 to 3, **characterized in that** the filter disk (11) arranged closer to the radiation source (3) is made of a material more susceptible to breaking than the filter disk (12) located further away from the radiation source (3).

5. The irradiation device (1) according to any one of claims 1 to 4, **characterized in that** the frame (6, 7) comprises cooling ribs (16) on its outer circumference.

6. The irradiation device according to any one of claims 1 to 5, **characterized in that** the irradiation device (1) comprises a fan (15) and the cooling ribs (16) are configured such that the free cross-section (17) between the cooling ribs (16) and a housing (2) of the irradiation device (1) is optimized with respect to the air capacity of the fan (15).

## Revendications

1. Dispositif d'irradiation (1) avec une source de rayons (3) et un filtre (5) disposé sur la trajectoire des rayons et comprenant deux plaques filtrantes (11, 12) transparentes qui sont globalement parallèles et qui sont retenues avec leurs bords périphériques dans un cadre (6, 7) composé d'un matériau bon conducteur de chaleur, les plaques filtrantes (11, 12) et le cadre (6, 7) délimitant une cavité fermée (13) grâce au fait qu'il est prévu un agent qui influe sélectivement sur le spectre des rayons, la cavité (13) étant conçue comme une chambre de pression, de sorte que lorsque le dispositif d'irradiation (1) fonctionne, il règne une surpression dans ladite cavité (13), et les plaques filtrantes (11, 12) étant en vitrocéramique ou en verre minéral,
**caractérisé en ce que** la plaque filtrante (11) plus proche de la source de rayons (3) est conçue comme un dispositif de sécurité, de sorte qu'elle se casse plus facilement que la plaque filtrante (12) plus éloignée de la source de rayons (3).

2. Dispositif d'irradiation (1) selon la revendication 1, **caractérisé en ce que** la plaque filtrante (11) plus proche de la source de rayons (3) présente un point de rupture.

3. Dispositif d'irradiation (1) selon la revendication 1 ou 2, **caractérisé en ce que** la plaque filtrante (11) plus proche de la source de rayons (3) est plus mince que la plaque filtrante (12) plus éloignée de la source de rayons (3).

4. Dispositif d'irradiation (1) selon l'une des revendications 1 à 3, **caractérisé en ce que** la plaque filtrante (11) plus proche de la source de rayons (3) se compose d'un matériau qui se casse plus facilement que la plaque filtrante (12) plus éloignée de la source de rayons (3).

5. Dispositif d'irradiation (1) selon l'une des revendications 1 à 4, **caractérisé en ce que** le cadre (6, 7) présente sur sa circonférence extérieure des nervures de refroidissement (16).

6. Dispositif d'irradiation (1) selon l'une des revendications 1 à 5, **caractérisé en ce qu'**il comprend un ventilateur (15) et les nervures de refroidissement (16) sont conçues pour que la section transversale libre (17) entre les nervures de refroidissement (16) et un boîtier (2) du dispositif d'irradiation (1) soit optimisée par rapport au débit d'air du ventilateur (15).
